# EUROPEAN PATENT SPECIFICATION

(11) **EP 2 705 027 B1**
(45) Date of publication and mention of the grant of the patent: **03.06.2015**
(21) Application number: 12721645.5
(22) Date of filing: 30.04.2012
(51) Int. Cl.: C07D 277/28, A61K 31/427, A61P 31/12

(54) **Amorphous solid salts of Cobicistat (GS-9350)**
Amorphe feste Salze von Cobicistat (GS-9350)
Sels solides et amorphes de Cobicistat (GS-9350)

(30) Priority: 02.05.2011 US 201161481509 P
(43) Date of publication of application: 12.03.2014
(73) Proprietor: Gilead Sciences, Inc., Foster City, CA 94404 (US)
(72) Inventor: KOZIARA, Joanna M., Foster City, CA 94404 (US); LUONG, Anne, Mississauga, Ontario L5V 3A1 (CA); SUJINO, Keiko, Foster City, CA 94404 (US); YU, Richard, Foster City, CA 94404 (US)
(74) Representative: Hallybone, Huw George
(86) International application number: PCT/US2012/035871
(87) International publication number: WO 2012/151165

(56) References cited:
- WO-A1-2008/103949
- WO-A2-2009/135179
- WO-A2-2010/115000

## Description

### Background of the Invention

International patent application publication number WO 2008/010921 describes compounds and pharmaceutical compositions that improve the pharmacokinetics of a co-administered drug by inhibiting cytochrome P450 monooxygenase. One such inhibitor is the compound of formula (I). 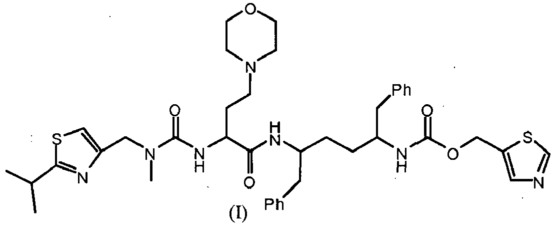 Unfortunately, the solid state properties of the compound of formula (I) make it difficult to handle and process on a large scale. For example, its low glass transition temperature, hygroscopicity, and lack of crystallinity, as well as its non free-flowing nature make it particularly difficult to process and to formulate (e.g. as a tablet).

International patent application publication number WO 2009/135,179 discusses the difficulties associated with processing the compound of formula (I) and describes combining the compound of formula (I) with solid carrier particles to improve the physical properties of the resulting solid material. Although the resulting free-flowing powder has high loading values for the compound of formula (I), acceptable physical and chemical stability, rapid drug release properties, and excellent compressibility, the inert carrier particles contribute to the overall weight and volume of the solid so that significantly more material is required in a formulation to achieve a given dose of the compound of formula (I). Accordingly, there is a need for solid forms of the compound of formula (I) that have the beneficial properties of the solids described in WO 2009/135,179, but lack the inert carrier particles that contribute to the weight and the volume of the solid. WO 2010/115,000 describes methods and intermediates that are useful for preparing a compound of formula (Ia), defined below.

### Summary of the Invention

The invention provides solid amorphous forms of the compound of formula (I) that have many of the beneficial properties of the materials discussed in international patent application publication number WO 2009/135,179, but lack the inert carrier particles.

Accordingly, in one embodiment, the invention provides an amorphous solid of the invention which is an amorphous solid of a salt of a compound of formula (I): 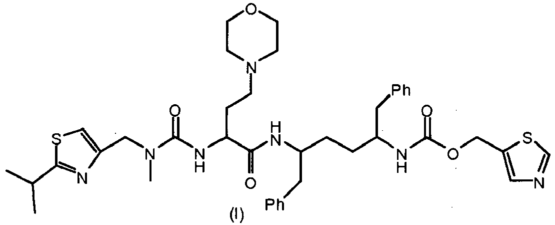 that is not coated on a silica particle.

In another embodiment the invention provides a pharmaceutical composition comprising an amorphous solid of the invention. In another embodiment the invention provides a tablet comprising an amorphous solid of the invention.

In another embodiment the invention provides a pharmaceutical composition comprising an amorphous solid of the invention; tenofovir disoproxil fumarate; emtricitabine; and elvitegravir.

In another embodiment the invention provides amorphous solids and pharmaceutical compositions for use in a method to inhibit the activity of cytochrome P-450 in an animal comprising administering an amorphous solid of the invention or a pharmaceutical composition of the invention to the animal (e.g. a mammal such as a human).

In another embodiment the invention provides amorphous solids in combination with a therapeutically effective amount of one or more therapeutic agents selected from the group consisting of HIV protease inhibiting compounds, HIV non-nucleoside inhibitors of reverse transcriptase, HIV nucleoside inhibitors of reverse transcriptase, HIV nucleotide inhibitors of reverse transcriptase, HIV integrase inhibitors, and CCR5 inhibitors, for use in a method for treating an HIV infection.

In another embodiment the invention provides an amorphous solid of the invention for use in medical therapy.

In another embodiment the invention provides an amorphous solid of the invention for use in the prophylactic or therapeutic treatment of an HIV infection.

In another embodiment the invention provides an amorphous solid of the invention for use in inhibiting the activity of cytochrome P-450.

In another embodiment the invention provides an amorphous solid of the invention for use in the preparation of a medicament for treating HIV infection in a mammal.

In another embodiment the invention provides an amorphous solid of the invention for use in the preparation of a medicament for inhibiting the activity of cytochrome P-450 in an animal.

In another embodiment the invention provides a method as described in claim 13 comprising: a) contacting a compound of formula (I) with a requisite acid in a first solvent, and b) adding a second solvent under conditions that allow a salt to form.

In another embodiment the invention provides a method as described in claim 14 comprising, spray drying a solution comprising a compound of formula (I) and a requisite acid under conditions that allow a salt to form.

Also disclosed is a method comprising adding a toluene solution of a compound formula (I): 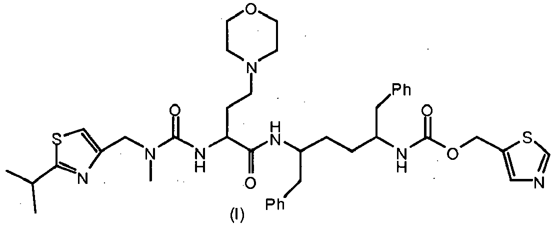 to heptanes to provide an amorphous solid.

In another embodiment the invention provides a method for preparing a pharmaceutical composition comprising combining the amorphous solid of the invention and a pharmaceutically acceptable excipient to provide the pharmaceutical composition.

In another embodiment the invention provides a method for preparing a pharmaceutical composition comprising: combining an amorphous solid of the invention, tenofovir disoproxil fumarate, emtricitabine, and elvitegravir to provide the pharmaceutical composition.

Also disclosed is a material prepared by a method described herein.

### Detailed Description of the Invention

It will be appreciated by those skilled in the art that compounds of formula (I) may exist in and be isolated in optically active and racemic forms. Some compounds may exhibit polymorphism. It is to be understood that the present invention encompasses any racemic, optically-active, polymorphic, or stereoisomeric form, or mixtures thereof, of a compound of formula (I), which possess the useful properties described herein, it being well known in the art how to prepare optically active forms (for example, by resolution of the racemic form by recrystallization techniques, by synthesis from optically-active starting materials, by chiral synthesis, or by chromatographic separation using a chiral stationary phase.

An "amorphous solid" as used herein includes solid materials that do not have a periodical three-dimensional pattern". For example, amorphous solids that lack crystallinity can be identified by XRPD analysis as illustrated herein. In one embodiment an amorphous solid is a free flowing, handible form (e.g. powder or solid). In another embodiment an amorphous solids excludes glassy, rubbery, and gum like materials including the formula I materials prepared in International Patent Application Publication Number WO 2008/010921.

In one embodiment the invention provides pharmaceutical compositions comprising an amorphous solid of the invention that can be administered to a mammalian host, such as a human patient, in a variety of forms adapted to the chosen route of administration (e.g. orally).

The compositions of the invention may include one or more pharmaceutically acceptable excipients. Excipients include but are not limited to substances that can serve as a vehicle or medium for an amorphous solid of the invention (e.g. a diluent or a carrier). They may be enclosed in hard or soft shell gelatin capsules, may be compressed into tablets, or may be incorporated directly with the food of the patient's diet. For oral therapeutic administration, the active compound may be combined with one or more excipients and used in the form of ingestible tablets, buccal tablets, troches, capsules, elixirs, suspensions, syrups, wafers, and the like. Such compositions and preparations will typically contain at least 0.1% of active compound. The percentage of the compositions and preparations may, of course, be varied and may conveniently be between about 2 to about 60% of the weight of a given unit dosage form. The amount of active compound in such therapeutically useful compositions is such that an effective dosage level will be obtained.

The tablets, troches, pills, capsules, and the like may also contain the following: binders such as hydroxypropyl cellulose, povidone, or hydroxypropyl methylcellulose; fillers, such as microcrystalline cellulose, pregelatinized starch, starch, mannitol, or lactose monohydrate; a disintegrating agent such as croscarmellose sodium, cross-linked povidone, or sodium starch glycolate; a lubricant such as magnesium stearate, stearic acid, or other metallic stearates; and a sweetening agent such as sucrose, fructose, lactose or aspartame or a flavoring agent such as peppermint, oil of wintergreen, or cherry flavoring may be added. When the unit dosage form is a capsule, it may contain, in addition to materials of the above type, a liquid carrier, such as a vegetable oil or a polyethylene glycol. Various other materials may be present as coatings or to otherwise modify the physical form of the solid unit dosage form. For instance, tablets, pills, or capsules may be coated with gelatin, polymers, wax, shellac or sugar and the like. Of course, any material used in preparing any unit dosage form will typically be pharmaceutically acceptable and substantially non-toxic in the amounts employed. In addition, the compositions of the invention may be incorporated into sustained-release preparations and devices.

The compositions of the invention can also be administered topically, e.g., transdermally, buccally, or sublingually. Accordingly, the invention also provides pharmaceutical compositions that are formulated for such routes of topical administration. Useful dosages of the compounds of formula (I) can be determined by comparing their *in vitro* activity, and *in vivo* activity in animal models. Methods for the extrapolation of effective dosages in mice, and other animals, to humans are known to the art.

The amount of a composition of the invention required for use in treatment will vary with the route of administration, the nature of the condition being treated and the age and condition of the patient and will be ultimately at the discretion of the attendant physician or clinician.

In general, however, a suitable dose of the compound of formula (I) will be in the range of from about 0.05 to about 100 mg/kg, e.g., from about 0.05 to about 50 mg/kg of body weight per day, preferably in the range of 0.05 to 10 mg/kg/day, most preferably in the range of 0.05 to 5 mg/kg/day.

The compound is conveniently formulated in unit dosage form; for example, containing about 5 to 500 mg, about 5 to 250 mg, or about 10 to 100 mg of the compound of formula (I). In one embodiment, the invention provides a composition comprising about 5, about 25, or about 100 mg of a compound of formula (I) formulated in a unit dosage, and one or more pharmaceutically acceptable excipients.

The ability of a compound of formula (I) to inhibit cytochrome P-450 can be evaluated as described in international patent application publication number WO 2008/010921.

### Combination Formulations

As discussed in international patent application publication number WO 2008/010921, the compound of formula (I) improves the pharmacokinetics of a co-administered drug, *e.g.,* by inhibiting cytochrome P-450 monooxygenase. Accordingly, in another embodiment, the pharmaceutical compositions of the invention can further comprise at least one additional therapeutic agent.

The additional therapeutic agent can be any agent having a therapeutic effect when used in combination with the compound of the present invention. For example, the additional therapeutic agent used in combination with the compound of formula (I) can be any agent that is accessible to oxidative metabolism by cytochrome P450 enzymes, especially cytochrome P450 monooxygenase, *e.g.,* 1A2, 2B6, 2C8, 2C19, 2C9, 2D6, 2E1, 3A4, 5, 7, etc.

In one example, the additional therapeutic agent can be any anti-viral agent, *e.g.,* anti-HIV, anti-HCV, etc., anti-bacterial agent, anti-fungal agent, immuno-modulator, *e.g.,* immunosuppressant, anti-neoplastic agent, chemotherapeutic agent, agents useful for treating cardiovascular conditions, neurological conditions, etc. In another example, the additional therapeutic agent can be any proton pump inhibitor, anti-epileptics, NSAID, oral hypoglycemic agent, angiotensin II receptor antagonist, sulfonylurea, beta blocker, antidepressant, antipsychotic, or anesthetic, or a combination thereof.

In another example, the additional therapeutic agent can be any 1) macrolide antibiotic, *e.g.,* clarithromycin, erythromycin, telithromycin, 2) anti-arrhythmic, *e.g.,* quinidine=>3-OH, 3) benzodiazepine, *e.g.,* alprazolam, diazepam=>3-OH, midazolam, triazolam, 4) immune modulator, *e.g.,* cyclosporine, tacrolimus (FK506), 5) HIV antiviral, *e.g.,* indinavir, nelfinavir, ritonavir, saquinavir, 6) prokinetic, *e.g.,* cisapride, 7) antihistamine, *e.g.,* astemizole, chlorpheniramine, terfenidine, 8) calcium channel blocker, *e.g.,* amlodipine, diltiazem, felodipine, lercanidipine, nifedipine, nisoldipine, nitrendipine, verapamil, 9) HMG CoA reductase inhibitor, *e.g.,* atorvastatin, cerivastatin, lovastatin, simvastatin, or 10) steroid 6beta-OH, *e.g.,* estradiol, hydrocortisone, progesterone, testosterone.

In another example, the additional therapeutic agent can be alfentanyl, aprepitant, aripiprazole, buspirone, cafergot, caffeine, TMU, cilostazol, cocaine, codeine- N-demethylation, dapsone, dextromethorphan, docetaxel, domperidone, eplerenone, fentanyl, finasteride, gleevec, haloperidol, irinotecan, LAAM, lidocaine, methadone, nateglinide, ondansetron, pimozide, propranolol, quetiapine, quinine, salmeterol, sildenafil, sirolimus, tamoxifen, paclitaxel, terfenadine, trazodone, vincristine, zaleplon, or zolpidem or a combination thereof.

In one specific embodiment, the invention provides a pharmaceutical composition comprising, 1) an amorphous solid of the invention, 2) at least one additional therapeutic agent selected from the group consisting of HIV protease inhibiting compounds, HIV non-nucleoside inhibitors of reverse transcriptase, HIV nucleoside inhibitors of reverse transcriptase, HIV nucleotide inhibitors of reverse transcriptase, HIV integrase inhibitors, non-nucleoside inhibitors of HCV, CCR5 inhibitors, and combinations thereof, and 3) a pharmaceutically acceptable excipient.

In another embodiment, the present invention provides pharmaceutical compositions comprising 1) an amorphous solid of the invention, 2) at least one additional therapeutic agent selected from the group consisting of amprenavir, atazanavir, fosamprenavir, indinavir, lopinavir, ritonavir, nelfinavir, saquinavir, tipranavir, brecanavir, darunavir, TMC-126, TMC-114, mozenavir (DMP-450), JE-2147 (AG1776), L-756423, RO0334649, KNI-272, DPC-681, DPC-684, GW640385X, DG17, PPL-100, DG35, AG 1859, capravirine, emivirine, delaviridine, efavirenz, nevirapine, (+)-calanolide A, etravirine, GW5634, DPC-083, DPC-961, DPC-963, MIV-150, TMC-120, TMC-278 (rilpivirine), BILR 355 BS, VRX 840773, UK-453061, RDEA806, zidovudine, emtricitabine, didanosine, stavudine, zalcitabine, lamivudine, abacavir, amdoxovir, elvucitabine, alovudine, MIV-210, Racivir (±-FTC), D-d4FC, phosphazide, fozivudine tidoxil, apricitibine AVX754, amdoxovir, KP-1461, and fosalvudine tidoxil (formerly HDP 99.0003), tenofovir disoproxil fumarate, adefovir dipivoxil, GS-9131, curcumin, derivatives of curcumin, chicoric acid, derivatives of chicoric acid, 3,5-dicaffeoylquinic acid, derivatives of 3,5-dicaffeoylquinic acid, aurintricarboxylic acid, derivatives of aurintricarboxylic acid, caffeic acid phenethyl ester, derivatives of caffeic acid phenethyl ester, tyrphostin, derivatives of tyrphostin, quercetin, derivatives of quercetin, S-1360, zintevir (AR-177), L-870812, L-870810, MK-0518 (raltegravir), elvitegravir, BMS-538158, GSK364735C, BMS-707035, MK-2048, BA 011, enfuvirtide, sifuvirtide, FB006M, TRI-1144, AMD-070, SP01A, BMS-488043, BlockAide/ CR, immunitin, benzimidazole derivatives, benzo-1,2,4-thiadiazine derivatives, phenylalanine derivatives, aplaviroc, vicriviroc, and maraviroc, cyclosporine, FK-506, rapamycin, paclitaxel, taxotere, clarithromycin, A-77003, A-80987, MK-639, saquinavir, VX-478, AG1343, DMP-323, XM-450, BILA 2011 BS, BILA 1096 BS, BILA 2185 BS, BMS 186,318, LB71262, SC-52151, SC-629 (N,N-dimethylglycyl-N-(2-hydroxy-3-(((4-methoxyphenyl)sulphonyl)(2-methylpropyl)amino)-1 -(phenylmethyl)propyl)-3-methyl-L-valinamide), KNI-272, CGP 53437, CGP 57813 and U-103017; and 3) a pharmaceutically acceptable excipient.

In another embodiment, the present invention provides pharmaceutical compositions comprising, 1) an amorphous solid of the invention, and 2) two or three additional therapeutic agents. For example, additional therapeutic agents selected from the classes of HIV protease inhibitors, HIV non-nucleoside inhibitors of reverse transcriptase, HIV nucleoside inhibitors of reverse transcriptase, HIV nucleotide inhibitors of reverse transcriptase, and HIV integrase inhibitors. The two or three additional therapeutic agents can be different therapeutic agents selected from the same class of therapeutic agents, or they can be selected from different classes of therapeutic agents.

In another embodiment, the invention provides pharmaceutical compositions that comprise a ternary combination of agents selected from an amorphous solid of the invention,/tenofovir disoproxil fumarate/emtricitabine, an amorphous solid of the invention/tenofovir disoproxil fumarate/elvitegravir, an amorphous solid of the invention/tenofovir disoproxil fumarate/efavrenz, an amorphous solid of the invention/tenofovir disoproxil fumarate/atazanavir, an amorphous solid of the invention/tenofovir disoproxil fumarate/darunavir, an amorphous solid of the invention/tenofovir disoproxil fumarate/raltegravir, an amorphous solid of the invention/tenofovir disoproxil fumarate/rilpivirine, an amorphous solid of the invention/emtricitabine/elvitegravir, an amorphous solid of the invention/emtricitabine/efavrenz, an amorphous solid of the invention/emtricitabine/atazanavir, an amorphous solid of the invention/emtricitabine/darunavir, an amorphous solid of the invention/emtricitabine/raltegravir, an amorphous solid of the invention/emtricitabine/rilpivirine, an amorphous solid of the invention/elvitegravir/efavrenz, an amorphous solid of the invention/elvitegravir/atazanavir, an amorphous solid of the invention/elvitegravir/darunavir, an amorphous solid of the invention/elvitegravir/raltegravir, an amorphous solid of the invention/elvitegravir/rilpivirine, an amorphous solid of the invention/efavrenz/atazanavir, an amorphous solid of the invention/efavrenz/darunavir, an amorphous solid of the invention/efavrenz/raltegravir, an amorphous solid of the invention/efavrenz/rilpivirine, an amorphous solid of the invention/atazanavir/darunavir, an amorphous solid of the invention/atazanavir/raltegravir, an amorphous solid of the invention/atazanavir/rilpivirine, an amorphous solid of the invention/darunavir/raltegravir, an amorphous solid of the invention/darunavir/rilpivirine, and an amorphous solid of the invention/raltegravir/rilpivirine.

In another embodiment, the invention provides pharmaceutical compositions that comprise a quaternary combination of agents selected from an amorphous solid of the invention/tenofovir disoproxil fumarate/emtricitabine/elvitegravir, an amorphous solid of the invention/tenofovir disoproxil fumarate/emtricitabine/efavrenz, an amorphous solid of the invention/tenofovir disoproxil fumarate/emtricitabine/atazanavir, an amorphous solid of the invention/tenofovir disoproxil fumarate/emtricitabine/darunavir, an amorphous solid of the invention/tenofovir disoproxil fumarate/emtricitabine/raltegravir, an amorphous solid of the invention/tenofovir disoproxil fumarate/emtricitabine/rilpivirine, an amorphous solid of the invention/tenofovir disoproxil fumarate/elvitegravir/efavrenz, an amorphous solid of the invention/tenofovir disoproxil fumarate/elvitegravir/atazanavir, an amorphous solid of the invention/tenofovir disoproxil fumarate/elvitegravir/darunavir, an amorphous solid of the invention/tenofovir disoproxil fumarate/elvitegravir/raltegravir, an amorphous solid of the invention/tenofovir disoproxil fumarate/elvitegravir/rilpivirine, an amorphous solid of the invention/tenofovir disoproxil fumarate/efavrenz/atazanavir, an amorphous solid of the invention/tenofovir disoproxil fumarate/efavrenz/darunavir, an amorphous solid of the invention/tenofovir disoproxil fumarate/efavrenz/raltegravir, an amorphous solid of the invention/tenofovir disoproxil fumarate/efavrenz/rilpivirine, an amorphous solid of the invention/tenofovir disoproxil fumarate/atazanavir/darunavir, an amorphous solid of the invention/tenofovir disoproxil fumarate/atazanavir/raltegravir, an amorphous solid of the invention/tenofovir disoproxil fumarate/atazanavir/rilpivirine, an amorphous solid of the invention/tenofovir disoproxil fumarate/darunavir/raltegravir, an amorphous solid of the invention/tenofovir disoproxil fumarate/darunavir/rilpivirine, an amorphous solid of the invention/tenofovir disoproxil fumarate/raltegravir/rilpivirine, an amorphous solid of the invention/GS-9131/emtricitabine/elvitegravir, an amorphous solid of the invention/emtricitabine/elvitegravir/efavrenz, an amorphous solid of the invention/emtricitabine/elvitegravir/atazanavir, an amorphous solid of the invention/emtricitabine/elvitegravir/darunavir, an amorphous solid of the invention/emtricitabine/elvitegravir/raltegravir, an amorphous solid of the invention/emtricitabine/elvitegravir/rilpivirine, an amorphous solid of the invention/emtricitabine/efavrenz/atazanavir, an amorphous solid of the invention/emtricitabine/efavrenz/darunavir, an amorphous solid of the invention/emtricitabine/efavrenz/raltegravir, an amorphous solid of the invention/emtricitabine/efavrenz/rilpivirine, an amorphous solid of the invention/emtricitabine/atazariavir/darunavir, an amorphous solid of the invention/emtricitabine/atazanavir/raltegravir, an amorphous solid of the invention/emtricitabine/atazanavir/rilpivirine, an amorphous solid of the invention/emtricitabine/darunavir/raltegravir, an amorphous solid of the invention/emtricitabine/darunavir/rilpivirine, an amorphous solid of the invention/emtricitabine/raltegravir/rilpivirine, an amorphous solid of the invention/elvitegravir/efavrenz/atazanavir, an amorphous solid of the invention/elvitegravir/efavrenz/darunavir, an amorphous solid of the invention/elvitegravir/efavrenz/raltegravir, an amorphous solid of the invention/elvitegravir/efavrenz/rilpivirine, an amorphous solid of the invention/elvitegravir/atazanavir/darunavir, an amorphous solid of the invention/elvitegravir/atazanavir/raltegravir, an amorphous solid of the invention/elvitegravir/atazanavir/rilpivirine, an amorphous solid of the invention/elvitegravir/darunavir/raltegravir, an amorphous solid of the invention/elvitegravir/darunavir/rilpivirine, an amorphous solid of the invention/elvitegravir/raltegravir/rilpivirine, an amorphous solid of the invention/efavrenz/atazanavir/darunavir, an amorphous solid of the invention/efavrenz/atazanavir/raltegravir, an amorphous solid of the invention/efavrenz/atazanavir/rilpivirine, an amorphous solid of the invention/efavrenz/darunavir/raltegravir, an amorphous solid of the invention/efavrenz/darunavir/rilpivirine, an amorphous solid of the invention/efavrenz/raltegravir/rilpivirine, an amorphous solid of the invention/atazanavir/darunavir/raltegravir, an amorphous solid of the invention/atazanavir/darunavir/rilpivirine, and an amorphous solid of the invention/darunavir/raltegravir/rilpivirine.

### Combination Methods of Treatment

In one embodiment, the compositions of the invention that comprise an amorphous solid of the invention can be used alone, e.g., for inhibiting cytochrome P450 monooxygenase. In another embodiment, the compositions of the invention can be used in combination with other active therapeutic ingredients or agents. Preferably, the other active therapeutic ingredients or agents are metabolized or accessible to the oxidative metabolism by cytochrome P450 enzymes, *e.g.,* monooxygenase enzymes such as 1A2, 2B6, 2C8, 2C19, 2C9, 2D6, 2E1, 3A4, 5, 7, etc., thereby reducing the amount or rate at which the other active therapeutic agent or ingredient is metabolized, whereby the pharmacokinetics of the other active therapeutic agent or ingredient is improved. Such improvements can include elevating the blood plasma levels of the other therapeutic agent or ingredient or maintaining a more therapeutically effective blood plasma level of the other therapeutic active agent or ingredient compared to blood plasma levels of the other therapeutic agent or ingredient administered without the compositions of the invention that comprise an amorphous solid of the invention.

Co-administration of an amorphous solid of the invention with one or more other active therapeutic agents generally refers to simultaneous or sequential administration of the amorphous solid of the invention and one or more other active therapeutic agents, such that therapeutically effective amounts of the amorphous solid of the invention and one or more other active therapeutic agents are both present in the body of the patient.

Co-administration includes administration of unit dosages of the amorphous solid of the invention before or after administration of unit dosages of one or more other active therapeutic agents, for example, administration of the amorphous solid of the invention within seconds, minutes, or hours of the administration of one or more other active therapeutic agents. For example, a unit dose of a compound of an amorphous solid of the invention can be administered first, followed within seconds or minutes by administration of a unit dose of one or more other active therapeutic agents. Alternatively, a unit dose of one or more other therapeutic agents can be administered first, followed by administration of a unit dose of amorphous solid of the invention within seconds or minutes. In some cases, it may be desirable to administer a unit dose of an amorphous solid of the invention first, followed, after a period of hours (e.g., 1 to 12 hours), by administration of a unit dose of one or more other active therapeutic agents. In other cases, it may be desirable to administer a unit dose of one or more other active therapeutic agents first, followed, after a period of hours (e.g., 1 to 12 hours), by administration of a unit dose of an amorphous solid of the invention.

In yet another embodiment, the present invention provides a composition of the invention that comprises an amorphous solid of the invention for use in a method for improving the pharmacokinetics of a drug which is metabolized by cytochrome P450 monooxygenase, comprising administering to a patient treated with said drug, a therapeutically effective amount of a composition of the invention that comprises an amorphous solid of the invention.

In yet another embodiment, the present application provides a composition of the invention that comprises an amorphous solid of the invention for use in a method for improving the pharmacokinetics of a drug which is metabolized by cytochrome P450 monooxygenase, comprising administering to a patient treated with said drug, a therapeutically effective amount of a composition of the invention that comprises an amorphous solid of the invention.

In yet another embodiment, the present application provides a composition of the invention that comprises an amorphous solid of the invention for use in a method for improving the pharmacokinetics of a drug which is metabolized by cytochrome P450 monooxygenase 3A, comprising administering to a patient treated with said drug, a composition of the invention that comprises an amorphous solid of the invention.

In yet another embodiment, the present application provides a composition of the invention that comprises an amorphous solid of the invention for use in a method for increasing blood plasma levels of a drug which is metabolized by cytochrome P450 monooxygenase, comprising administering to a patient treated with said drug, a composition of the invention that comprises an amorphous solid of the invention.

In yet another embodiment, the present application provides a composition of the invention that comprises an amorphous solid of the invention for use in a method for increasing blood plasma levels of a drug which is metabolized by cytochrome P450 monooxygenase 3A, comprising administering to a patient treated with said drug, a composition of the invention that comprises an amorphous solid of the invention.

In yet another embodiment, the present application provides a composition of the invention that comprises an amorphous solid of the invention for use in a method for inhibiting cytochrome P450 monooxygenase 3A in a patient comprising administering to a patient in need thereof an amount of a composition of the invention that comprises an amorphous solid of the invention, effective to inhibit cytochrome P450 monooxygenase 3A.

In yet another embodiment, the present application provides a composition of the invention that comprises an amorphous solid of the invention in combination with a therapeutically effective amount of one or more additional therapeutic agents selected from the group consisting of HIV protease inhibiting compounds, HIV non-nucleoside inhibitors of reverse transcriptase, HIV nucleoside inhibitors of reverse transcriptase, HIV nucleotide inhibitors of reverse transcriptase, HIV integrase inhibitors, and CCR5 inhibitors, for use in a method for treating an HIV infection.

In yet another embodiment, the present application provides a composition of the invention that comprises an amorphous solid of the invention in combination with a therapeutically effective amount of one or more additional therapeutic agents selected from the group consisting of amprenavir, atazanavir, fosamprenavir, indinavir, lopinavir, ritonavir, nelfinavir, saquinavir, tipranavir, brecanavir, darunavir, TMC-126, TMC-114, mozenavir (DMP-450), JE-2147 (AG1776), L-756423, RO0334649, KNI-272, DPC-681, DPC-684, and GW640385X, DG17, PPL-100, DG35, AG 1859, capravirine, emivirine, delaviridine, efavirenz, nevirapine, (+) calanolide A, etravirine, GW5634, DPC-083, DPC-961, DPC-963, MIV-150, TMC-120, TMC-278 (rilpivirine), efavirenz, BILR 355 BS, VRX 840773, UK-453061, RDEA806, zidovudine, emtricitabine, didanosine, stavudine, zalcitabine, lamivudine, abacavir, amdoxovir, elvucitabine, alovudine, MIV-210, racivir (±-FTC), D-d4FC, emtricitabine, phosphazide, fozivudine tidoxil, apricitibine (AVX754), amdoxovir, KP-1461, fosalvudine tidoxil (formerly HDP 99.0003), tenofovir disoproxil fumarate, adefovir dipivoxil, curcumin, derivatives of curcumin, chicoric acid, derivatives of chicoric acid, 3,5-dicaffeoylquinic acid, derivatives of 3,5-dicaffeoylquinic acid, aurintricarboxylic acid, derivatives of aurintricarboxylic acid, caffeic acid phenethyl ester, derivatives of caffeic acid phenethyl ester, tyrphostin, derivatives of tyrphostin, quercetin, derivatives of quercetin, S-1360, zintevir (AR-177), L-870812, L-870810, MK-0518 (raltegravir), elvitegravir, BMS-538158, GSK364735C, BMS-707035, MK-2048, and BA 011, enfuvirtide, sifuvirtide, FB006M, and TRI-1144, AMD-070, an entry inhibitor, SP01A, BMS-488043, BlockAide/ CR, a G6PD and NADH-oxidase inhibitor, immunitin, aplaviroc, vicriviroc, maraviroc, PRO-140, INCB15050, PF-232798 (Pfizer), CCR5mAb004, BAS-100, SPI-452, REP 9, SP-01A, TNX-355, DES6, ODN-93, ODN-112, VGV-1, PA-457 (bevirimat), Ampligen, HRG214, Cytolin, VGX-410, KD-247, AMZ 0026, CYT 99007A-221 HIV, DEBIO-025, BAY 50-4798, MDX010 (ipilimumab), PBS 119, ALG 889, and PA-1050040 (PA-040), for use in a method for treating an HIV infection.

In yet another embodiment, the present application provides a composition of the invention that comprises an amorphous solid of the invention in combination with a therapeutically effective amount of one or more additional therapeutic agents selected from the group consisting of pegylated rIFN-alpha 2b, pegylated rIFN-alpha 2a, rIFN-alpha 2b, rIFN-alpha 2a, consensus IFN alpha (infergen), reaferon, intermax alpha, r-IFN-beta, infergen + actimmune, IFN-omega with DUROS, locteron, albuferon, rebif, Oral interferon alpha, IFNalpha-2b XL, AVI-005, PEG-Infergen, and pegylated IFN-beta, rebetol, copegus, viramidine (taribavirin), NM-283, valopicitabine, R1626, PSI-6130 (R1656), HCV-796, BILB 1941, XTL-2125, MK-0608, NM-107, R7128 (R4048), VCH-759, PF-868554, GSK625433, SCH-503034 (SCH-7), VX-950 (telaprevir), BILN-2065, BMS-605339, ITMN-191, MX-3253 (celgosivir), UT-231B, IDN-6556, ME 3738, LB-84451, MitoQ, benzimidazole derivatives, benzo-1,2,4-thiadiazine derivatives, phenylalanine derivatives, A-831, A-689, zadaxin, nitazoxanide (alinea), BIVN-401 (virostat), PYN-17 (altirex), KPE02003002, actilon (CPG-10101), KRN-7000, civacir, GI-5005, ANA-975, XTL-6865, ANA 971, NOV-205, tarvacin, EHC-18, NIM811, DEBIO-025, VGX-410C, EMZ-702, AVI 4065, Bavituximab, Oglufanide, and VX-497 (merimepodib), for use in a method for treating an HCV infection.

### Specific embodiments of the invention

Specific embodiments identified herein are for illustration; they do not in any way exclude other embodiments of the invention.

In one embodiment of the invention the amorphous solid of the compound of formula (I) is a salt.

Disclosed is an amorphous solid of the compound of formula (I) is a spray dried foam that is not a salt.

In one embodiment of the invention the amorphous solid of the compound of formula (I) is isolated by precipitation from a solution.

In one embodiment of the invention, the compound of formula (I) is enriched with a stereoisomer of formula (Ia): 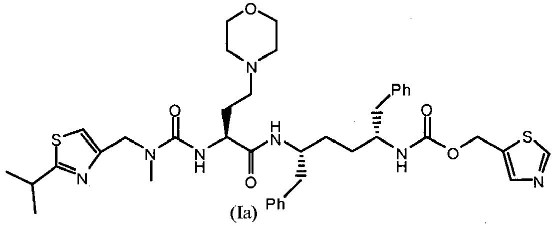 which is (3*R*,6*R*,9*S*)-12-methyl-13-[2-(1-methylethyl)-4-thiazolyl]-9-[2- (4-morpholinyl)ethyl]-8,11-dioxo-3,6-bis(phenylmethyl)-2,7,10,12-tetraazatridecanoic acid, 5-thiazolylmethyl ester. The compound of formula (Ia) is referred to in the Examples herein below as Compound **1.** In one embodiment the compound of formula (I) has an enriched concentration of 85 ± 5% of the stereoisomer of formula (Ia). In another embodiment the compound of formula (I) has an enriched concentration of 90 ± 5% of the stereoisomer of formula (Ia). In another embodiment the compound of formula (I) has an enriched concentration of 95 ± 2% of the stereoisomer of formula (Ia). In another embodiment the compound of formula (I) has an enriched concentration of 99 ± 1% of the stereoisomer of formula (Ia). In another embodiment the compound of formula (I) is the pure the stereoisomer of formula (Ia).

In one embodiment the invention provides an amorphous solid of a compound of formula I or a salt thereof that is not coated on a fumed silica particle (i.e. coated in the pores or on the surface of a fumed silica particle).

In one embodiment the invention provides an amorphous solid of a compound of formula I or a salt thereof that is not coated on a silica particle.

In one embodiment the invention provides an amorphous solid of a compound of formula I or a salt thereof that is not coated on a solid carrier (e.g. kaolin, bentonite, hectorite, colloidal magnesium-aluminum silicate, silicon dioxide, magnesium trisilicate, aluminum hydroxide, magnesium hydroxide, magnesium oxide and talc).

In one embodiment the invention provides an amorphous solid of a compound of formula I or a salt thereof that is not coated on a solid carrier as described in international patent application publication number WO 2009/135,179.

The invention will now be illustrated by the following non-limiting Examples.

### Examples

### Example 1. Preparation of a Representative Composition of the Invention

Compound **1** (3.4 g, 4.38 mmol) was dissolved in toluene (12.2 g). The solution was charged to heptanes (122 g) maintaining about 5 °C. The resulting slurry was agitated at about 5 °C, then filtered. Solids were rinsed forward with heptanes, then dried under vacuum at ambient temperature. Dry product was obtained as an off-white solid (2.77 g, 3.57 mmol, 81% yield, 98.0% AN by HPLC). ¹H NMR (400 MHz, DMSO-*d₆*) δ = 1.30 (d, *J* = 6.8 Hz, 6H), 1.33-1.42 (m, 4H), 1.57-1.65 (m, 1 H), 1.70-1.79 (m, 1H), 2.13-2.35 (m, 6H), 2.60 (d, *J* = 6.8, 2H), 2.65 (t, *J* = 6.4, 2H), 2.89 (s, 3H), 3.23 (ddd, *J* = 6.8, 6.8, 14, 1H), 3.50 (t, *J* = 4 Hx, 4H), 3.58-3.68 (m, 1H), 3.88-3.90 (m, 1H), 4.08 (dd, *J* = 7.2, 12.8 Hz, 1H), 4.47 (s, 2H), 5.17 (s, 2H), 6.61 (d, *J =* 7.6 Hz, 1H), 7.08-7.23 (m, 12H), 7.51 (d, *J* = 8.8 Hz, 1H), 9.08 (s, 1H).

### Compound 1 Solid:

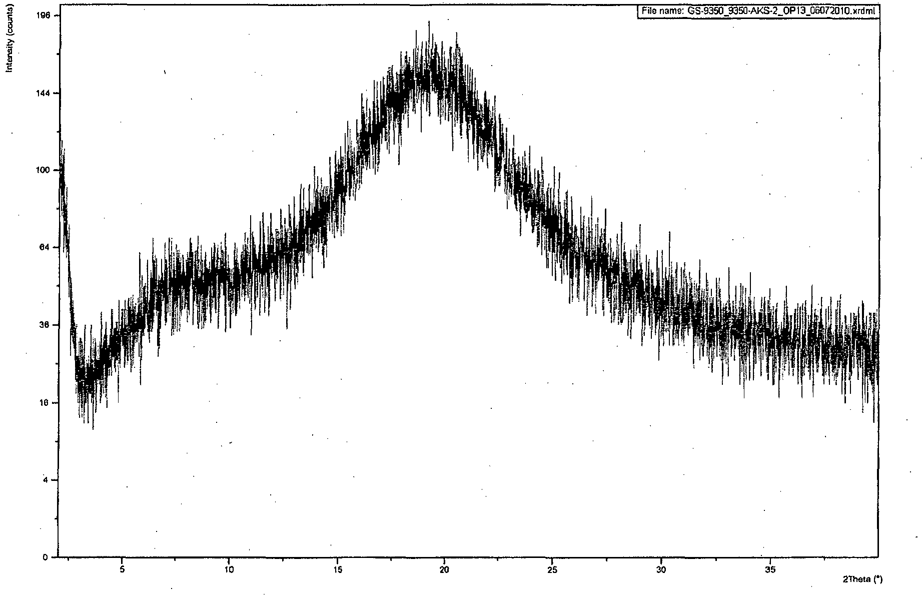

### Example 2. Preparation of a Representative Citric Acid Salt of the Invention

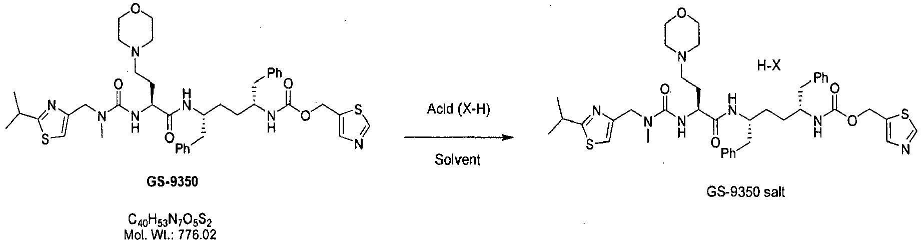

Citric acid (0.5 g, 2 equiv.) was dissolved in isopropyl acetate (15 g) at reflux. Compound **1** (1 g, 1.29 mmol, 1 equiv.) was charged and the mixture was heated to reflux for about 15 minutes. The solution was adjusted to ambient. Heptanes (15 g) were charged to precipitate the salt. The product was filtered, rinsed forward with heptanes and dried under vacuum at 40 °C to give an off-white solid (1.43 g, 0.97 mmol, 75% yield, 94.1% AN by HPLC). ¹H NMR (400 MHz, DMSO-d₆) δ = 1.30 (d, *J* = 7.2 Hz, 6H), 1.32-1.47 (m, 4H), 1.62-1.72 (m, 1 H), 1.75-1.87 (m, 1H), 2.30-2.66 (m, 10H), 2.66 (dd, 4H, *J* = 15.2, 38.3 Hz)*, 2.89 (s, 3H), 3.20-3.27 (m, 1H), 3.53-3.68 (m, 4H), 3.88-3.99 (m, 1H), 4.08-4.13 (m, 1H), 4.47 (s, 2H), 5.16 (s, 2H), 6.60 (d, *J =* 7.2, 1H), 7.09-7.23 (m, 12H), 7.56 (d, *J* = 8.8, 1H), 7.87 (s, 1 H), 9.08 (s, 1H). As shown in the following plot, the product (top line) was contaminated with crystalline citric acid (lower line).
*citrate, 3.6 equiv.

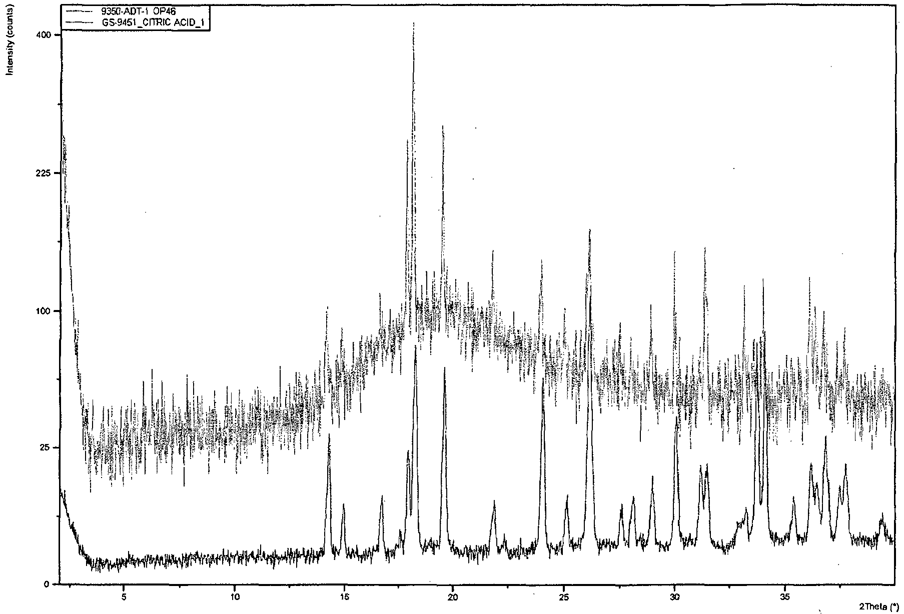

### Example 3. Preparation of a Representative Citric Acid Salt of the Invention

A solution of Compound **1** (2 g, 2.58 mmol) and citric acid (0.4 g, 0.21 mmol, 0.8 equiv.) in dichloromethane (10.5 g) was charged to methyl *tert*-butylether (182 g) drop wise at ambient temperature. The resultant slurry was agitated at ambient temperature. Solids were filtered and dried under vacuum at ~ 40 °C to yield white solids (1.64 g, 1.69 mmol, 66% yield, 93.3% AN by HPLC). ¹H NMR (400 MHz, DMSO-*d₆*) δ = 1.30 (d, *J* = 7.2 Hz, 6H), 1.32-1.47 (m, 4H), 1.62-1.72 (m, 1 H), 1.75-1.87 (m, 1H), 2.30-2.66 (m, 10H), 2.66 (dd, 4H, *J=* 15.2, 38.3 Hz)*, 2.89 (s, 3H), 3.20-3.27 (m, 1H), 3.53-3.68 (m, 4H), 3.88-3.99 (m, 1H), 4.08-4.13 (m, 1H), 4.47 (s, 2H), 5.16 (s, 2H), 6.60 (d, *J* = 7.2, 1H), 7.09-7.23 (m, 12H), 7.56 (d, *J* = 8.8, 1H), 7.87 (s, 1H), 9.08 (s, 1H).
*citrate, 1 equiv.

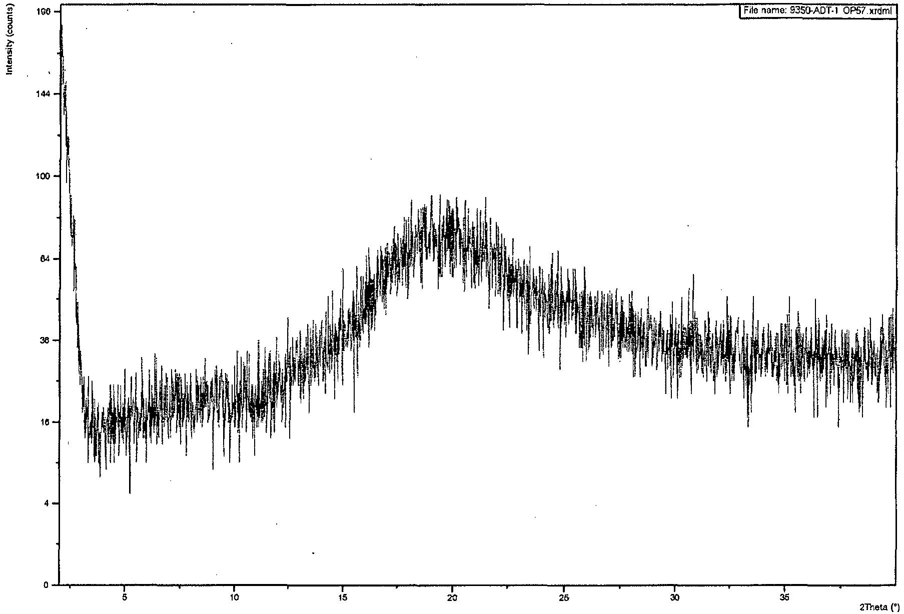

### Example 4. Preparation of a Representative Tartrate Salt of the Invention

A mixture of Compound **1** (1.25 g, 1 equiv.) and *L*-tartaric acid (0.5 g, 2 equiv.) in isopropyl alcohol (3.9 g) was agitated at about 45 °C for about 15 minutes. The resultant solution was adjusted to ambient temperature, then methyl *tert*-butylether (45 g) was charged to yield a slurry. The product was filtered, rinsed forward with heptanes and dried under vacuum at 40 °C to give an off-white solid (quantitative yield, 95.5% AN). ¹H NMR (400 MHz, DMSO-*d₆*) δ = 1.30 (d, *J* = 7.2, 6H), 1.32-1.47 (m, 4H), 1.63-1.70 (m, 1H), 1.75-1.82 (m, 1H), 2.24-2.52 (m, 6H), 2.60-2.72 (m, 4H), 2.89 (s, 3H), 3.18-3.28 (m, 1H), 3.53-3.68 (m, 4H), 3.88-3.99 (m, 1H), 4.07-4.12 (m, 1H), 4.28 (s, 2H)*, 4.47 (s, 2H), 5.16 (s, 2H), 6.61 (d, *J* = 7.2, 1H), 7.09-7.23 (m, 12H), 7.55 (d, *J* = 8.8, 1 H), 7.87 (s, 1H), 9.08 (s, 1H). * tartrate, 2 equiv.

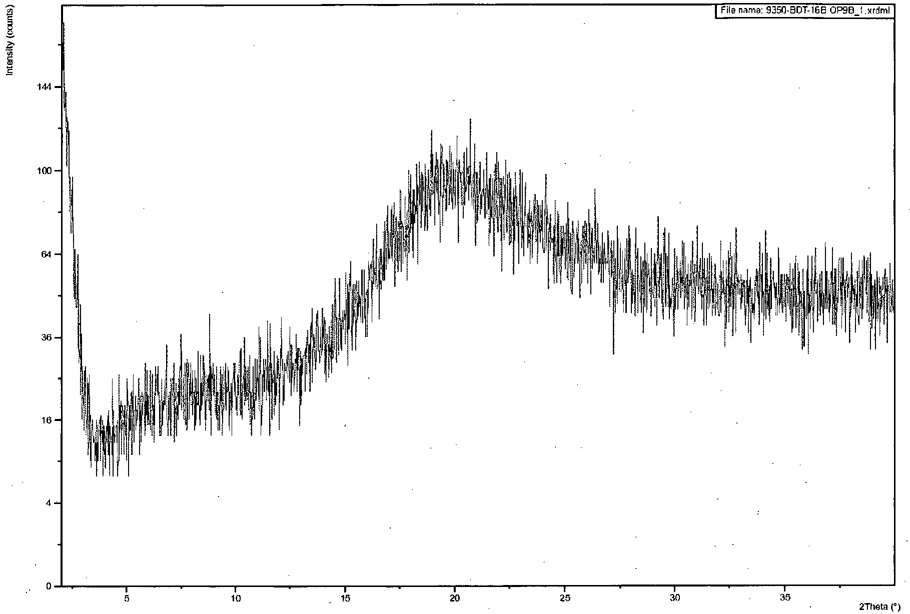

### Example 5. Preparation of a Representative Oxalate Salt of the Invention

To a solution of oxalic acid (0.28 g, 3.22 mmol, 2.4 equiv.) in isopropyl acetate (11 g) at ambient temperature, a solution of Compound **1** (1 g, 1.29 mmol, 1.0 equiv.) in isopropyl acetate (3 g) was charged drop-wise at ambient temperature followed by heptane (10 g). The resultant slurry was agitated at ambient temperature for about 30 minutes The product was filtered and rinsed forward with heptanes and dried under vacuum at 40 °C to give a light yellow solid (1.27 g, 99% yield, 95.4% AN by HPLC). ¹H NMR: (400 MHz, DMSO-*d₆*) δ = 1.30 (d, *J* = 7.2 Hx, 6H), 1.30-1.50 (m, 4H), 1.85-1.95 (m, 1H), 1.95-2.05 (m, 1H), 2.62-2.67 (m, 4H), 2.93 (s, 3H), 2.95-3.01 (m, 2H), 4.07-3.20 (m, 4H), 3.20-2.37 (m, 1H), 3.60-3.70 (m, 1H), 3.75-3.89 (m, 4H), 3.90-3.98 (m, 1H), 4.50 (dd, *J* = 16.4, 22.4 Hz, 2H), 5.17 (s, 2H), 6.57 (d, *J* = 7.2 Hz, 1H), 7.05-7.25 (m, 12H), 7.71 (d, *J =* 8.4 Hz, 1H), 7.87 (s, 1H), 9.08 (s, 1H). Oxalate (2 equiv based on the mole ratio of input acid.).

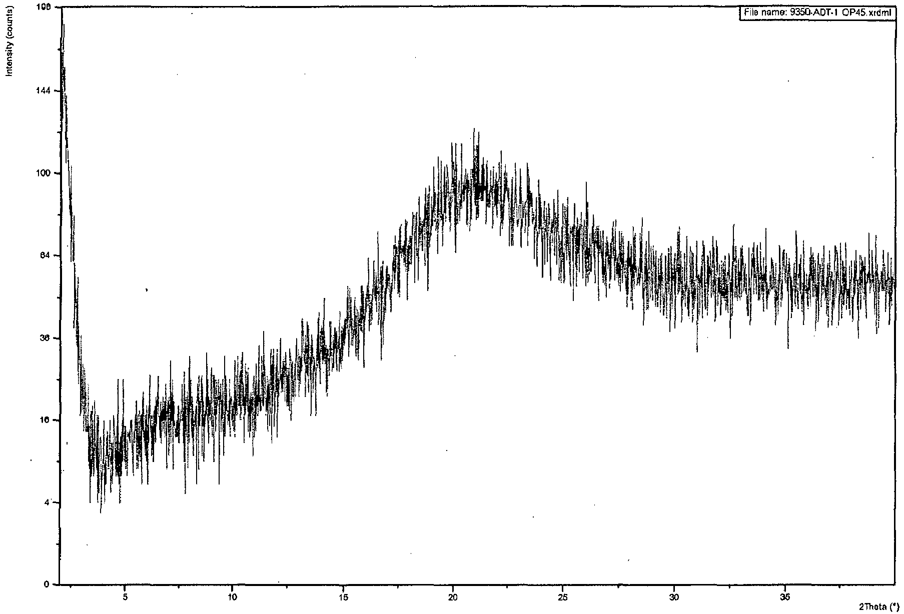

### Example 6. Preparation of a Representative Fumarate Salt of the Invention

Fumaric acid (0.3 g, 2.58 mmol, 2 equiv.) was dissolved in isopropyl alcohol (5 g) at reflux. Compound **1** (1 g, 1.29 mmol, 1 equiv.) was charged. The resulting solution was concentrated under vacuum in a bath set at about 40 °C. Isopropyl acetate (25 g) and heptane (30 g) were charged. The resulting slurry was agitated at ambient temperature for about 30 minutes Solids were filtered and dried under vacuum at 40 °C. An off-white solid was obtained (1.02 g, 1.01 mmol as bis fumarate, 78%, 94.0% AN by HPLC). ¹H NMR (400 MHz, DMSO-*d₆*) δ = 1.3 (d, 6H), 1.3-1.5 (m, 4H), 1.6-1.7 (m, 1H), 1.7-1.8 (m, 1H), 2.2-2.5 (m, 6H), 2.6-2.77 (m, 4H), 2.9 (s, 3H), 3.2-3.3 (m, 1H), 3.5-3.7 (m, 5H), 3.9-4.0 (m, 1H), 4.1-4.2 (m, 1H), 4.5 (s, 2H), 5.2 (s, 2H), 6.6 (m, 1H), 6.6 (S, 2H)*, 7.1-7.3 (m, 12H), 7.6 (d, 1H), 7.9 (s, 1H), 9.1 (s, 1H). *Fumarate, 2 equiv.

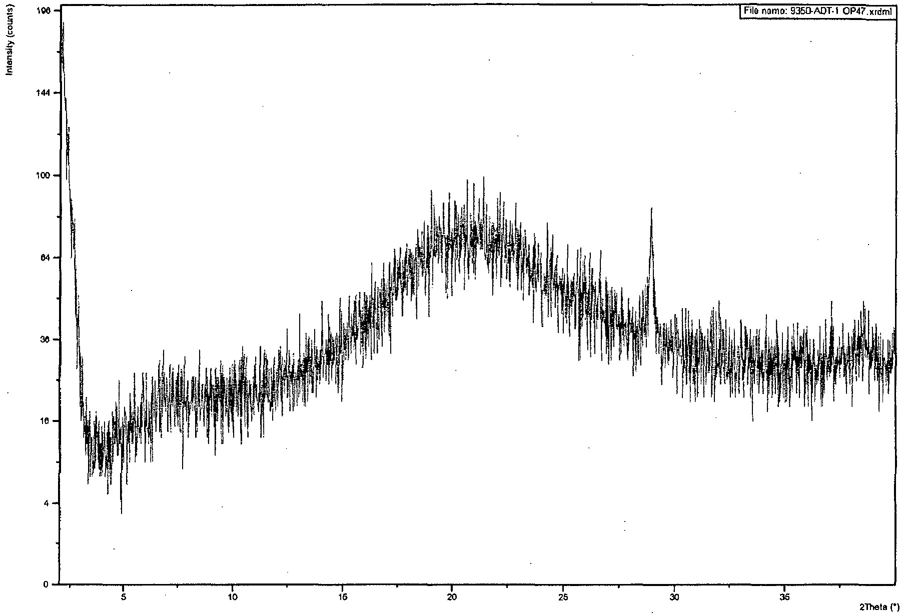

### Example 7. Preparation of a Representative Hydrochloric Acid Salt of the Invention

Compound **1** (8.35 g, 10.8 mmol, 1 equiv.) was dissolved in 1.25 M HCl in ethanol (6.8 mL, HCl 8.5 mmol, 0.8 equiv.). The resulting solution was charged to methyl *tert-*butylether (84 g). The resulting mixture was concentrated under vacuum at about 40 °C. Methyl *tert*-butylether (80 g) was charged. The mixture was agitated at ambient. The product was filtered, rinsed forward with methyl *tert*-butylether, then dried under vacuum at about 40 °C, to provide an off-white solid (7.31 g, 9.0 mmol, 83% yield, 96.8% AN). ¹H NMR (400 MHz, DMSO-*d₆*) δ =1.30 (d, *J* = 6.8, 6H), 1.33-1.47 (m, 4H), 1.96-2.12 (m, 2H), 2.24-2.52 (m, 6H), 2.61-2.71 (m, 4H), 2.82 (s, 3H), 2.95-3.09 (m, 2H), 3.23 (ddd, *J* = 6.8, 6.8, 14 Hz, 1H), 3.30-3.40 (m, 4H), 3.60-3.68 (m, 1H), 3.76-3.88 (m, 2H), 3.88-3.98 (m, 3H), 4.16-4.22 (m, 1H), 4.49 (dd, *J* = 16.4, 22.4 Hz,, 2H), 5.16 (s, 2H), 6.57 (d, *J* = 6.8 Hz, 1H), 7.09-7.24 (m, 11H), 7.84-7.87 (m, 2H), 9.09 (s, 1H), 11.1 (br, 1H). Titration: hydrochloride, 1 equiv.

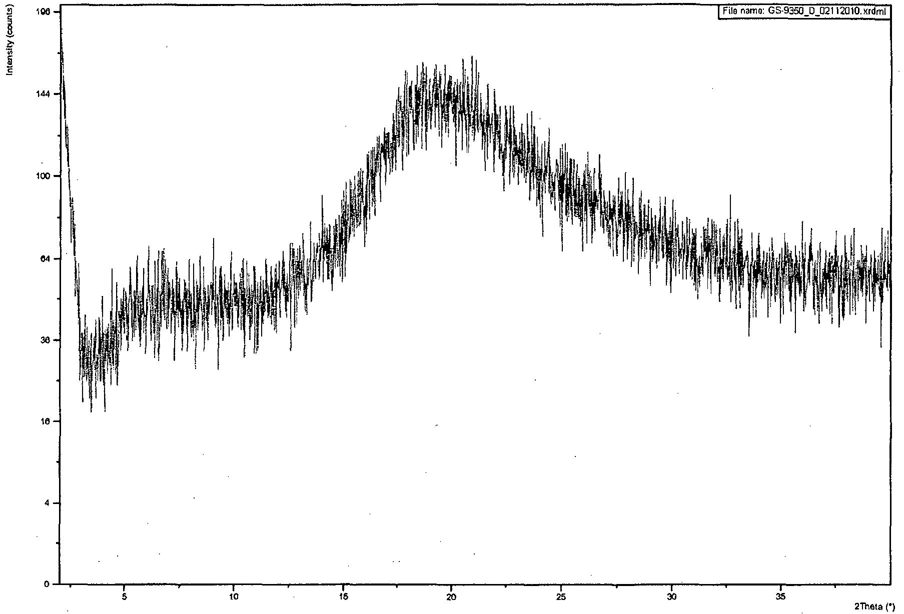

### Example 8. Preparation of a Representative Lactate Salt of the Invention

Compound **1** (2.7 g, 3.48 mmol, 1 equiv.) and lactic acid (85% aqueous solution, 0.37 g, 3.49 mmol, 1 equiv. ) were dissolved in ethanol (10.5 g). The resultant solution was concentrated under vacuum, and coevaporated with toluene. The concentrate was dissolved in toluene (9 g), and charged to heptanes at about 5 °C. The resulting slurry was agitated at about 5 °C, then filtered and rinsed forward with heptanes. The product was dried at about 40 °C to afford light brown solid (2.81 g, 3.24 mmol, 93% yield, 97.5% AN). ¹H NMR (400 MHz, DMSO-*d₆*) δ = 1.24 (d, *J* = 6.8 Hx, 3H)*, 1.30 (d, *J* = 7.3 Hz, 6H), 1.34-1.41 (m, 4H), 1.62 (dt, *J* = 6.8, 20.8, 1 H), 1.76 (dt, *J* = 6.6, 20, 1 H), 2.15-2.34 (m, 6H), 2.60-2.66 (m, 4H), 2.89 (s, 3H), 3.23 (ddd, *J* = 7.2, 13.6, 20.8 Hz), 3.51 (t, *J* = 4.4, 3H), 3.60-3.68 (m, 1H), 3.90-3.98 (m, 1H), 4.04 (dd, *J* = 6.8, 13.8 Hz, 1H)*, 4.09 (dd, *J =* 6.8, 12.8 Hz, 1 H), 4.47 (s, 2H), 5.17 (s, 2H), 7.07-7.24 (m, 12H), 7.53 (d, *J* = 8.4 Hz, 1H), 7.87 (s, 1H), 9.08 (s, 1H). * lactate, 1 equiv.

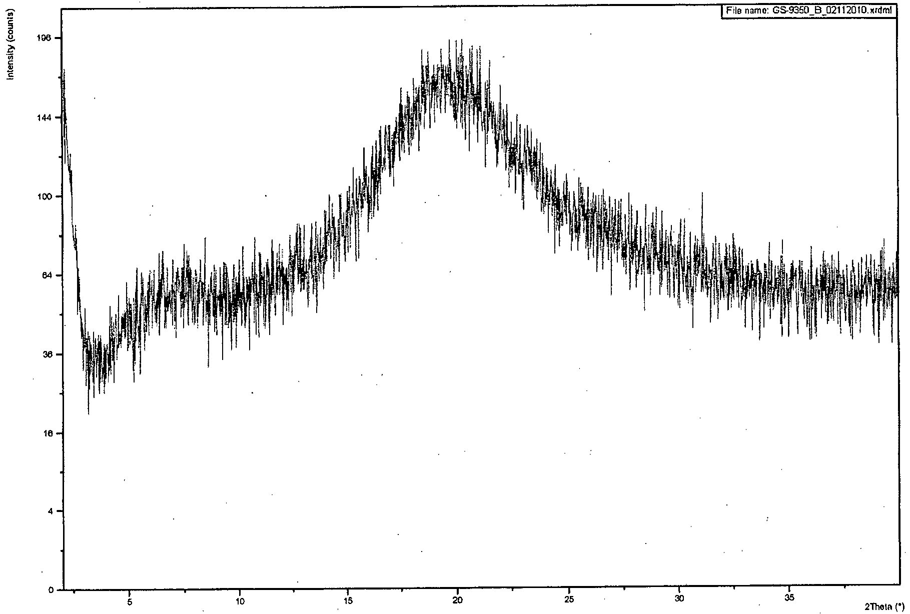

### Example 9. Preparation of a Representative Phosphate Salt of the Invention

Compound **1** (4.70 g, 6.06 mmol, 1 equiv.) and H₃PO₄ (0.72 g, 85%, 6.24 mmol, 1 equiv.) was dissolved in isopropyl alcohol (10 g) at about 40 °C. The mixture was adjusted to ambient temperature. Heptanes (80 g) were charged. The resulting slurry was agitated at ambient. The product was filtered and rinsed forward with heptanes, then dried under vacuum at about 40 °C to afford a light brown solid (5.2 g, 5.74 mmol, 95% yield). ¹H NMR (400 MHz, DMSO-*d₆*) δ = 1.30 (d, *J* = 7.2, 6H), 1.3 5-1.40 (m, 4H), 1.62-1.70 (m, 1H), 1.75-1.83 (m, 1H), 2.26-2.48 (m, 6H), 2.60 (d, *J* = 6.8 Hz, 2H), 2.65 (t, *J* = 5.6 Hz, 2H), 2.89 (s, 3 H), 3.23 (ddd, J = 7.2, 7.2, 20.8 Hz, 1H), 3.56 (m, 4H), 3.60-3.67 (m, 1H), 3.88-3.98 (m, 1H), 4.07-4.12 (m, 1H), 4.47 (s, 2H), 5.16 (s, 2H), 6.62 (d, *J* = 7.4, 1H), 7.10-7.23 (m, 12H), 7.56 (d, *J* = 8.4, 1H), 7.87 (s, 1H), 9.08 (s, 1H). Titration: phosphate, 1 equiv.

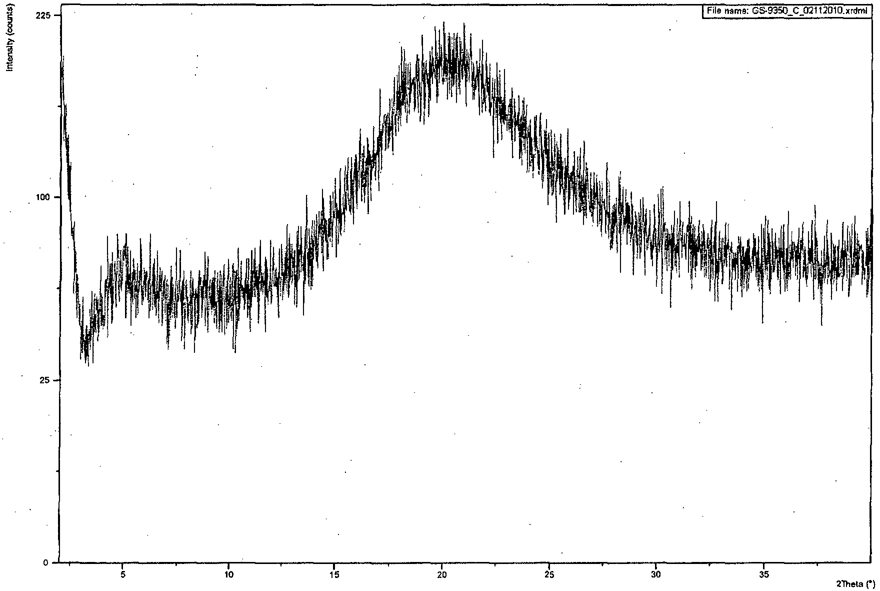

### Example 10. Preparation of a Representative Sulfate Salt of the Invention

Compound **1** (3.47 g, 4.47 mmol, 1 equiv) and conc. H₂SO₄ (250 □L, 4.46 mmol, 1 equiv.) were dissolved in isopropyl alcohol (10.5 g) at ambient temperature. The mixture was concentrated to dryness under vacuum. The residue was dissolved in a mixture of dimethoxyethane (60 g) and isopropanol (5 g). The solution was charged to methyl *tert-*butylether (175 g), and the resulting slurry was agitated at ambient. The product was filtered, rinsed forward with methyl *tert*-butylether and dried under vacuum at 40 °C to give an off-white solid (2.63 g, 3.01 mmol, 67% yield). ¹H NMR (400 MHz, DMSO-*d₆*) δ = 1.30 (d, *J* = 7.2, 6H), 1.34-1.45 (m, 4H), 1.83-2.02 (m, 1H), 2.64 (dd, *J* = 6.4 16.0 Hz, 4H), 2.91 (s, 3H), 2.97-3.11 (m, 4H), 3.23 (ddd, *J =* 6.4, 6.4, 13 .6 Hz, 1H), 3.38 (dd, *J* = 11.6, 22 Hz, 1H), 3.60-3.68 (m, 3H), 3.90-4.00 (m, 3H), 4.18 (dd, *J* = 8.0, 13,2 Hz, 1H), 4.46 (d, *J* = 16.4 Hz, 1H), 4.53 (d, *J* = 16.4 Hz, 1H), 5.17 (s, 2H), 6.54 (d, *J* = 7.8, 1 H), 7.10-7.24 (m, 12H), 7.70 (d, *J =* 8.4 Hz, 1 H), 7.87 (s, 1H), 9.09 (s, 1H), 9.61 (br, 1H). Titration: sulfate, 1 equiv.

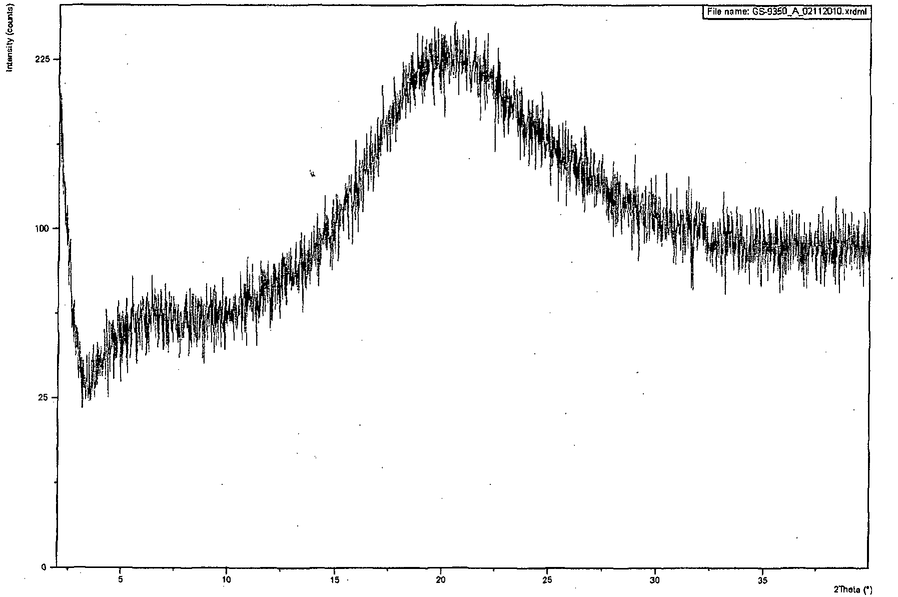

### Example 11. Preparation of a Representative Malate Salt of the Invention

Compound **1** (2.0 g, 2.58 mmol, 1 equiv.) and (S)-(-) malic acid (348 mg, 2.60 mmol, 1 equiv.) were dissolved in dichloromethane (6.7 g) at ambient temperature. The resulting solution was charged to methyl *tert*-butylether (75 g). Solids were filtered, and rinsed forward with methyl *tert*-butylether. The product was dried under vacuum at 40 °C to give an off-white solid (1.28 g, 1.41 mmol, 54% yield, 98.7% AN). ¹H NMR (400 MHz, DMSO-*d₆*) δ = 1.30 (d, *J* = 7.2, 6H), 1.34-1.41 (m, 4H), 1.64 (ddd, *J* = 7.2, 7.2, 13.6, 1H), 1.76 (ddd, *J* = 6.8, 6.8, 13.6 Hz), 2.20-2.45 (m, 7H), 2.55-2.67 (m, 5H), 2.89 (s, 3H), 3.23 (ddd, *J* = 7.2, 7.2, 14.0 Hz, 1H), 3.54 (t, *J* = 4.0 Hz, 4H), 3.58-3.68 (m, 1 H), 3.90-3.98 (m, 1H), 4.09 (dd, *J* = 7.2, 12.8, 1 H), 4.21 (dd, *J =* 5.6, 7.6, 1H)*, 4.47 (s, 2H), 5.16 (s, 2H), 6.61 (d, *J* = 6.8 Hz, 1H), 7.23-7.09 (m, 12H), 7.54 (d, *J* = 8.4 Hz, 1H), 7.87 (s, 1 H), 9.08 (s, 1 H). * malate, 1 equiv.

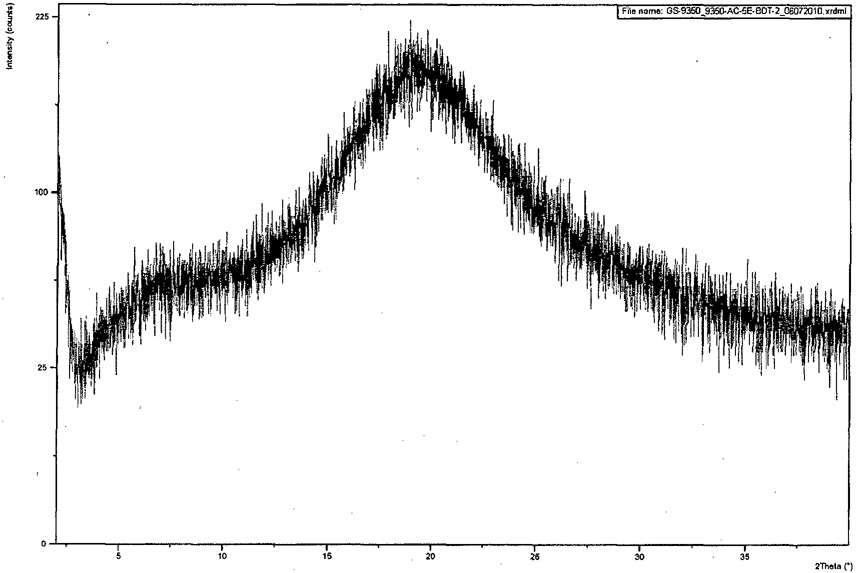

### Example 12. Determination of Glass Transition Temperatures

**Glass transition temperatures for the solids prepared in Examples 1-11 are shown in the following table.** Modulated differential scanning calorimetry (mDSC) was used to characterize salts of the invention. Solid samples were placed in hermetically sealed aluminum pan with a pinhole. Modulation amplitude of ± 0.8°C with a period of 60 sec was applied to the sample heated at 2 °C/min under dried nitrogen purge using TA instruments (New Castle, DE, USA) model 1000. Heat-cool-heat cycles were applied to allow for moisture evaporation during first heating cycle and to erase thermal history of the sample, which allowed for the determination of the intrinsic glass transition temperature (Tg) in dry state.

### Glass transition temperatures for representative salts of Compound 1

| **Salt Form** | **Tg (°C) First heating** | **Tg (°C) Second heating** |
|---|---|---|
| xinafoate | 49 | 51 |
| gentisate | 62 | 62 |
| phosphate | 66 | 69 |
| sulfate | 71 | 71 |
| citrate | 59 | 60 |
| tosylate | 32 | 46 |
| tartarate | 44 | 66 |
| bromide | 68 | 68 |
| dichloroacetate | ND | 34 |
| naphtalenesulfate | 58 | 58 |
| camphorosulfonate | 66 | 66 |
| nicotinate | 19.6 | 36 |
| lactate | 2 | 17 |
| hippurate | 31 | 44 |
| ethanesulfonate | -0.23 | 37 |
| malonate | 14 | 36 |
| succinate | 16 | 35 |
| fumarate | 37 | 51 |
| ketoglutarate | 24 | 42 |
| benzoate | 14 | 23 |
| besylate | 30 | 52 |
| edisylate | 33 | 93 |
| saccharinate | 37 | 56 |
| ascorbate | 42 | 60 |
| hydrochloride | 34 | 59 |
| maleate | 24 | 37 |
| oxalate | 34 | 46 |

### Example 13. Determination of Hygroscopic Properties

Hygroscopicity of salts of invention were measured by placing 20-50 mg of the sample in an uncapped scintillation vial. The vial was stored at ambient temperature in a Pyrex brand desiccator (VWR, International, CT). The humidity within the desiccator was controlled at 55% RH and 75% RH using saturated aqueous solutions of magnesium nitrite and sodium chloride, respectively. The relative humidity was confirmed using a digital hygrometer pen (VWR International, CT). An empty scintillation vial was also placed in the desiccator as a control. The weight gain for all the samples was determined after 24 hours; it was not determined whether equilibrium was reached after that period. Visual observation of phase transition was also noted.

Hygroscopicity data for representative salts of compound 1 is provided in the following Table.

### Hygroscopicity at room temperature

| **Salt Form** | **55% RH** | | **75% RH** | |
|---|---|---|---|---|
| | **% weight gain** | **Phase transition** | **% weight gain** | **Phase transition** |
| xinafoate | 0.29 | No | 1.97 | No |
| gentisate | 2.17 | No | 5.2 | No |
| phosphate | 4.52 | No | 7.9 | Partial |
| sulfate | 4.60 | Partial | 10.3 | Yes |
| citrate | 3.17 | No | 6.5 | Partial |
| tosylate | 3.11 | Partial | 6.5 | Yes |
| tartarate | 3.23 | No | 7.6 | Partial |
| bromide | 3.24 | No | 6.0 | Yes |
| dichloroacetate | 3.36 | Yes | 7.3 | Yes |
| naphtalenesulfate | 2.80 | No | 5.8 | Yes |
| camphorosulfonate | 2.99 | No | 5.9 | Yes |
| nicotinate | 2.07 | Yes | ND | Yes |
| lactate | ND | ND | 5.76 | Yes |
| hippurate | 3.37 | Partial | 5.61 | Yes |
| ethanesulfonate | 8.78 | Yes | 16.2 | Yes |
| malonate | 3.43 | Yes | 6.18 | Yes |
| succinate | 3.95 | Yes | 6.09 | Yes |
| fumarate | 2.11 | No | 6.02 | Yes |
| ketoglutarate | 3.27 | Yes | 5.23 | Yes |
| benzoate | 1.99 | Yes | 3.63 | Yes |
| besylate | 1.83 | Partial | 6.59 | Yes |
| edisylate | 6.01 | Yes | 10.7 | Yes |
| saccharinate | 1.89 | No | 4.09 | Yes |
| ascorbate | 3.23 | No | 7.56 | Yes |
| hydrochloride | 4.32 | Partial | 6.92 | Yes |
| maleate | 4.41 | Yes | 6.67 | Yes |
| oxalate | 4.38 | Partial | 9.36 | Yes |

### Example 14. Preparation of Representative Salts of the Invention

A salt of Compound **1** was dissolved in acetone at target concentration of 20% w/w and spry dried using GEA-Niro SDMicro™ Spray Dryer. Solution feeding rate ranged from 3-5 g/ml with atomizing gas pressure of 0.6-1 bar and drying gas rate of approximately 24 kg/hr. Drying gas temperature was maintained at about 45-50 °C and outlet temperature ranged between 40-45 °C.
Glass transition and hygroscopicity of the spray dried salts of the compound of invention are shown below.

| **API** | **Tg (°C)** | **Hygroscopicity** | | | |
|---|---|---|---|---|---|
| | | **Weight gain at 55% RH (%)** | **Phase transition** | **Weight gain at 75 % RH (%)** | **Phase transition** |
| Compound **1** gentisate | 61 | 3.1 | no | 5.6 | partial |
| Compound **1** xinafoate | 42 | 2.6 | no | 3.3 | no |
| Compound **1** tartarate | 67 | 5.8 | no | 6.3 | yes |
| Compound **1** phosphate | 92 | 4.7 | no | 8.0 | yes |
| Compound **1** fumarate | 31 | 3.2 | no | 6.0 | yes |
| Compound **1** saccharinate | 36.4 | nd | no | 4.0 | yes |
| Compound **1** chloride | 35 | 7.8 | partial | 9.4 | yes |

### Example 15. Representative Formulations of the Invention

The following illustrate representative pharmaceutical dosage forms, containing an amorphous solid of the compound of formula I ('Compound X'), for therapeutic or prophylactic use in humans.

| (i) Tablet 1 | mg/tablet |
|---|---|
| Compound X | 100.0 |
| Lactose | 77.5 |
| Povidone | 15.0 |
| Croscarmellose sodium | 12.0 |
| Microcrystalline cellulose | 92.5 |
| Magnesium stearate | 3.0 |
| | 300.0 |

| (ii) Tablet 2 | mg/tablet |
|---|---|
| Compound X | 20.0 |
| Microcrystalline cellulose | 410.0 |
| Starch | 50.0 |
| Sodium starch glycolate | 15.0 |
| Magnesium stearate | 5.0 |
| | 500.0 |

| (iii) Capsule | mg/capsule |
|---|---|
| Compound X | 10.0 |
| Colloidal silicon dioxide | 1.5 |
| Lactose | 465.5 |
| Pregelatinized starch | 120.0 |
| Magnesium stearate | 3.0 |
| | 600.0 |

| (iv) Injection 1 (1 mg/ml) | mg/ml |
|---|---|
| Compound X | 1.0 |
| Dibasic sodium phosphate | 12.0 |
| Monobasic sodium phosphate | 0.7 |
| Sodium chloride | 4.5 |
| 1.0 N Sodium hydroxide solution (pH adjustment to 7.0-7.5) | q.s. |
| Water for injection | q.s. ad 1 mL |

| (v) Injection 2 (10 mg/ml) | mg/ml |
|---|---|
| Compound X | 10.0 |
| Monobasic sodium phosphate | 0.3 |
| Dibasic sodium phosphate | 1.1 |
| Polyethylene glycol 400 | 200.0 |
| 01 N Sodium hydroxide solution (pH adjustment to 7.0-7.5) | q.s. |
| Water for injection | q.s. ad 1 mL |

| (vi) Aerosol | mg/can |
|---|---|
| Compound X | 20.0 |
| Oleic acid | 10.0 |
| Trichloromonofluoromethane | 5,000.0 |
| Dichlorodifluoromethane | 10,000.0 |
| Dichlorotetrafluoroethane | 5,000.0 |

The above formulations may be obtained by conventional procedures well known in the pharmaceutical art.

## Claims

1. An amorphous solid of a salt of a compound of formula (I): 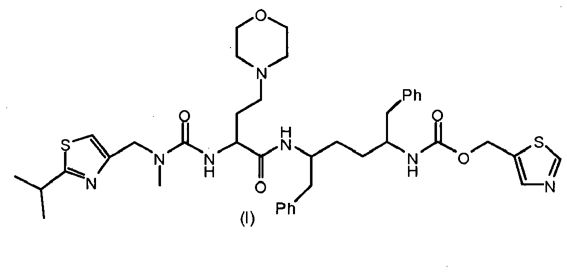 that is not coated on a silica particle, wherein the salt is (i) a citrate, tartrate, oxalate, fumarate, hydrochloride, lactate, phosphate, sulfate, or malate salt, or (ii) a xinafoate, tosylate, bromide, dichloroacetate, naphtalenesulfate, camphorosulfonate, nicotinate, hippurate, ethanesulfonate, malonate, succinate, ketoglutarate, benzoate, besylate, edisylate, saccharinate, ascorbate, or maleate salt.

2. The amorphous solid of claim 1 that has a glass transition temperature of at least about 40 °C, optionally of at least about 50 °C.

3. The amorphous solid of any one of claims 1-2 wherein the compound of formula (I) is a compound of formula (Ia):

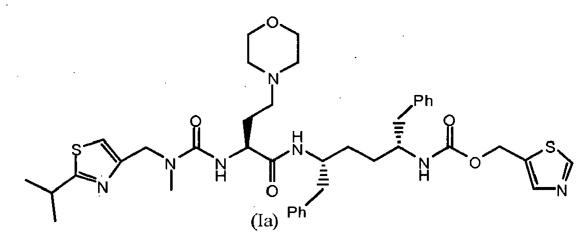

4. A pharmaceutical composition comprising an amorphous solid as described in any one of claims 1-3 and a pharmaceutically acceptable excipient.

5. A tablet comprising an amorphous solid as described in any one of claims 1-3.

6. A pharmaceutical composition comprising an amorphous solid as described in any one of claims 1-3; tenofovir disoproxil fumarate; emtricitabine; and elvitegravir.

7. An amorphous solid as described in any of claims 1-3 for use in a method to inhibit the activity of cytochrome P-450 in an animal.

8. An amorphous solid as described in any one of claims 1-3 in combination with a therapeutically effective amount of one or more therapeutic agents selected from the group consisting of HIV protease inhibiting compounds, HIV non-nucleoside inhibitors of reverse transcriptase, HIV nucleoside inhibitors of reverse transcriptase, HIV nucleotide inhibitors of reverse transcriptase, HIV integrase inhibitors, and CCR5 inhibitors, for use in a method for treating an HIV infection.

9. An amorphous solid as described in any one of claims 1-3 for use in medical therapy.

10. An amorphous solid as described in any one of claims 1-3 for use in the prophylactic or therapeutic treatment of an HIV infection.

11. An amorphous solid as described in any one of claims 1-3 for use in the preparation of a medicament for treating HIV infection in a mammal.

12. A method for the preparation of an amorphous solid of a salt of a compound of formula (I) according to claim 1 comprising: a) contacting a compound formula (I): 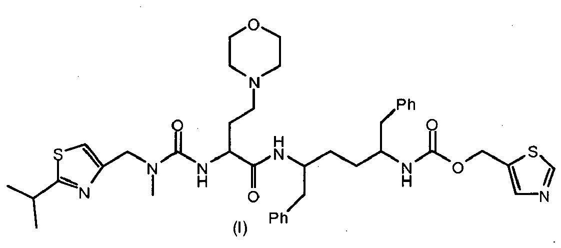 with a requisite acid in a first solvent, and b) adding a second solvent under conditions that allow a salt to form.

13. A method for the preparation of an amorphous solid of a salt of a compound of formula (I) according to claim 1 comprising, spray drying a solution comprising a compound of formula (I): 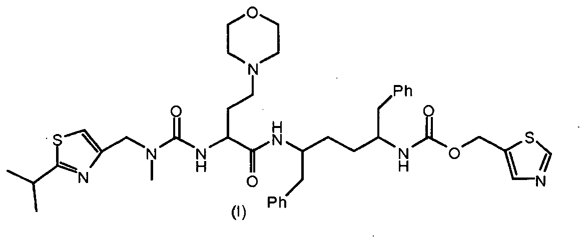 and a requisite acid under conditions that allow a salt to form.

14. A method for preparing a pharmaceutical composition comprising combining the amorphous solid as described in any one of claims 1-3 and a pharmaceutically acceptable excipient to provide the pharmaceutical composition.

15. A method for preparing a pharmaceutical composition comprising: combining an amorphous solid as described in any one of claims 1-3, tenofovir disoproxil fumarate, emtricitabine, and elvitegravir to provide the pharmaceutical composition.

## Patentansprüche

1. Amorpher Feststoff eines Salzes einer Verbindung der Formel (I): 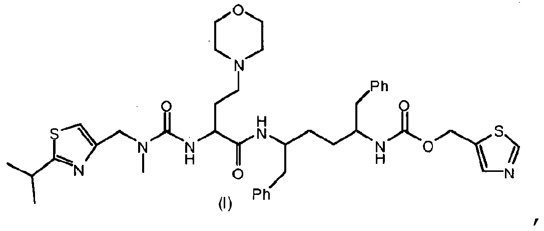 das nicht auf einem Kieselgelpartikel aufgetragen ist, wobei es sich bei dem Salz um (i) ein Citrat-, Tartrat-, Oxalat-, Fumarat-, Hydrochlorid-, Lactat-, Phosphat-, Sulfat- oder Maleatsalz oder (ii) ein Xinafoat-, Tosylat-, Bromid-, Dichloracetat-, Naphthalinsulfat-, Camphorosulfonat-, Nicotinat-, Hippurat-, Ethansulfonat-, Malonat-, Succinat-, Ketoglutarat-, Benzoat-, Besylat-, Edisylat-, Saccharinat-, Ascorbat- oder Maleatsalz handelt.

2. Amorpher Feststoff nach Anspruch 1 mit einer Glasübergangstemperatur von mindestens etwa 40°C, gegebenenfalls mindestens etwa 50°C.

3. Amorpher Feststoff nach Anspruch 1 oder 2, wobei es sich bei der Verbindung der Formel (I) um eine Verbindung der Formel (Ia) handelt:

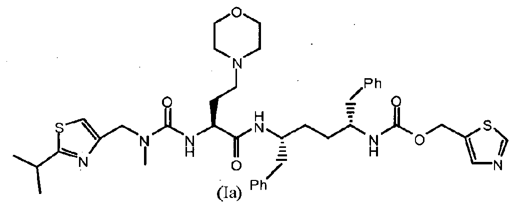

4. Pharmazeutische Zusammensetzung, umfassend einen amorphen Feststoff nach einem der Ansprüche 1-3 und einen pharmazeutisch unbedenklichen Exzipienten.

5. Tablette, umfassend einen amorphen Feststoff nach einem der Ansprüche 1-3.

6. Pharmazeutische Zusammensetzung, umfassend einen amorphen Feststoff nach einem der Ansprüche 1-3, Tenofovirdisoproxilfumarat, Emtricitabin und Elvitegravir.

7. Amorpher Feststoff nach einem der Ansprüche 1-3 zur Verwendung bei einem Verfahren zur Inhibierung der Aktivität von Cytochrom-P-450 in einem Tier.

8. Amorpher Feststoff nach einem der Ansprüche 1-3 in Kombination mit einer therapeutisch wirksamen Menge eines oder mehrerer therapeutischer Mittel ausgewählt aus der Gruppe bestehend aus HIV-Protease hemmenden Verbindungen, nichtnukleosidischen Inhibitoren der reversen Transkriptase von HIV, nukleosidischen Inhibitoren der reversen Transkriptase von HIV, nukleotidischen Inhibitoren der reversen Transkriptase von HIV, HIV-integrase Inhibitoren und CCRS-Inhibitoren zur Verwendung in einem Verfahren zur Behandlung einer HIV-Infektion.

9. Amorpher Feststoff nach einem der Ansprüche 1-3 zur Verwendung in der medizinischen Therapie.

10. Amorpher Feststoff nach einem der Ansprüche 1-3 zur Verwendung bei der prophylaktischen oder therapeutischen Behandlung einer HIV-Infektion.

11. Amorpher Feststoff nach einem der Ansprüche 1-3 zur Verwendung bei der Herstellung eines Medikaments zur Behandlung einer HIV-Infektion in einem Säugetier.

12. Verfahren zur Herstellung eines amorphen Feststoffs eines Salzes einer Verbindung der Formel (I) nach Anspruch 1, bei dem man: a) eine Verbindung der Formel (I): 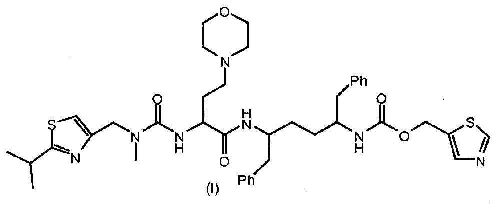 mit einer erforderlichen Säure in einem ersten Lösungsmittel in Kontakt bringt und b) ein zweites Lösungsmittel unter Bedingungen, die die Bildung eines Salzes ermöglichen, zugibt.

13. Verfahren zur Herstellung eines amorphen Feststoffs eines Salzes einer Verbindung der Formel (I) nach Anspruch 1, bei dem man eine eine Verbindung der Formel (I): 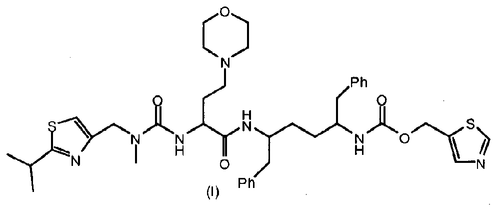 und eine erforderliche Säure umfassende Lösung unter Bedingungen, die die Bildung eines Salzes ermöglichen, sprühtrocknet.

14. Verfahren zur Herstellung einer pharmazeutischen Zusammensetzung, bei dem man den amorphen Feststoff nach einem der Ansprüche 1-3 und einen pharmazeutisch unbedenklichen Exzipienten zur Bereitstellung der pharmazeutischen Zusammensetzung kombiniert.

15. Verfahren zur Herstellung einer pharmazeutischen Zusammensetzung, bei dem man einen amorphen Feststoff nach einem der Ansprüche 1-3, Tenofovirdisoproxilfumarat, Emtricitabin und Elvitegravir zur Bereitstellung der pharmazeutischen Zusammensetzung kombiniert.

## Revendications

1. Solide amorphe d'un sel d'un composé de formule (I) : 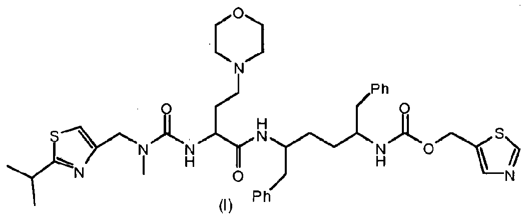 qui n'est pas revêtu sur une particule de silice, dans lequel le sel est (i) un sel de citrate, tartrate, oxalate, fumarate, chlorhydrate, lactate, phosphate, sulfate ou malate, ou (ii) un sel de xinafoate, tosylate, bromure, dichloroacétate, naphtalènesulfate, camphresulfonate, nicotinate, hippurate, éthane-sulfonate, malonate, succinate, cétoglutarate, benzoate, bésylate, édisylate, saccharinate, ascorbate, ou maléate.

2. Solide amorphe selon la revendication 1, qui possède une température de transition vitreuse d'au moins environ 40°C, éventuellement d'au moins environ 50°C.

3. Solide amorphe selon l'une quelconque des revendications 1-2, dans lequel le composé de formule (I) est un composé de formule (Ia) :

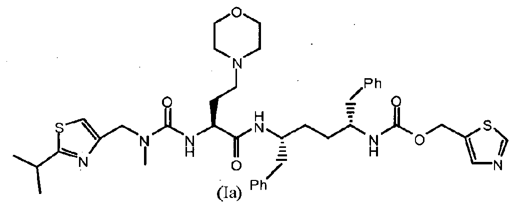

4. Composition pharmaceutique comprenant un solide amorphe tel que décrit selon l'une quelconque des revendications 1-3, et un excipient pharmaceutiquement acceptable.

5. Comprimé comprenant un solide amorphe tel que décrit selon l'une quelconque des revendications 1-3.

6. Composition pharmaceutique comprenant un solide amorphe tel que décrit selon l'une quelconque des revendications 1-3 ; du fumarate de ténofovir disoproxil ; de l'emtricitabine ; et de l'elvitégravir.

7. Solide amorphe tel que décrit selon l'une quelconque des revendications 1-3, pour une utilisation dans une méthode destinée à inhiber l'activité du cytochrome P-450 chez un animal.

8. Solide amorphe tel que décrit selon l'une quelconque des revendications 1-3, en combinaison avec une quantité thérapeutiquement efficace d'un ou plusieurs agents thérapeutiques choisis dans le groupe constitué par les composés inhibiteurs de la protéase du VIH, les inhibiteurs non nucléosidiques de la transcriptase inverse du VIH, les inhibiteurs nucléosidiques de la transcriptase inverse du VIH, les inhibiteurs nucléotidiques de la transcriptase inverse du VIH, les inhibiteurs de l'intégrase du VIH, et les inhibiteurs du CCR5, pour une utilisation dans une méthode destinée à traiter une infection par le VIH.

9. Solide amorphe tel que décrit selon l'une quelconque des revendications 1-3, pour une utilisation en thérapie médicale.

10. Solide amorphe tel que décrit selon l'une quelconque des revendications 1-3, pour une utilisation dans le traitement prophylactique ou thérapeutique d'une infection par le VIH.

11. Solide amorphe tel que décrit selon l'une quelconque des revendications 1-3, pour une utilisation dans la préparation d'un médicament destiné au traitement d'une infection par le VIH chez un mammifère.

12. Méthode de préparation d'un solide amorphe d'un sel d'un composé de formule (I) selon la revendication 1, comprenant : a) la mise en contact d'un composé de formule (I) : 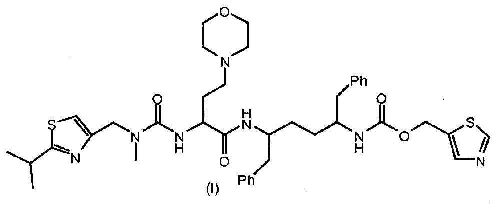 avec un acide requis dans un premier solvant, et b) l'addition d'un deuxième solvant dans des conditions permettant la formation d'un sel.

13. Méthode de préparation d'un solide amorphe d'un sel d'un composé de formule (I) selon la revendication 1, comprenant le séchage par pulvérisation d'une solution comprenant un composé de formule (I) : 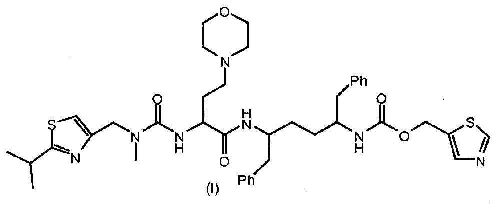 et d'un acide requis dans des conditions permettant la formation d'un sel.

14. Méthode de préparation d'une composition pharmaceutique, comprenant la combinaison du solide amorphe tel que décrit selon l'une quelconque des revendications 1-3 et d'un excipient pharmaceutiquement acceptable, afin de fournir la composition pharmaceutique.

15. Méthode de préparation d'une composition pharmaceutique, comprenant la combinaison d'un solide amorphe tel que décrit selon l'une quelconque des revendications 1-3, de fumarate de ténofovir disoproxil, d'emtricitabine, et d'elvitégravir, afin de fournir la composition pharmaceutique.
